# EUROPEAN PATENT SPECIFICATION

(11) **EP 3 638 348 B1**
(45) Date of publication and mention of the grant of the patent: **16.10.2024**
(21) Application number: 18833379.3
(22) Date of filing: 30.08.2018
(51) Int. Cl.: A61M 5/32, A61M 5/50, A61M 5/315

(54) **SAFETY SYRINGE**
SICHERHEITSSPRITZE
SERINGUE DE SÉCURITÉ

(43) Date of publication of application: 22.04.2020
(73) Proprietor: Jiangsu Caina Medical Co., Ltd, Zhutang Town Jiangyin City Jiangsu 214425 (CN)
(72) Inventor: LU, Jun, Jiangyin City, Jiangsu 214425 (CN); ZHAO, Lijie, Jiangyin City, Jiangsu 214425 (CN); NI, Lin, Jiangyin City, Jiangsu 214425 (CN); CHEN, Tianzhu, Jiangyin City, Jiangsu 214425 (CN)
(74) Representative: Proi World Intellectual Property GmbH
(86) International application number: PCT/CN2018/103110
(87) International publication number: WO 2020/042049

(56) References cited:
- WO-A1-2011/088731
- WO-A1-2011/088731
- WO-A1-2017/079811
- CN-A- 101 152 589
- CN-A- 102 971 033
- CN-A- 103 764 208
- CN-A- 105 188 813
- CN-A- 105 709 309
- CN-A- 106 668 989
- US-A1- 2007 185 458
- US-A1- 2010 179 486
- US-A1- 2010 262 119

## Description

### TECHNICAL FIELD

The present invention relates to the field of automatically retracting syringes, sometimes called safety syringes, having a needle that retracts into the body of the device at the completion of the fluid injection process to prevent accidental contact with the needle.

### BACKGROUND

A variety of safety syringe devices are known in the literature and commercial market. Such devices typically have a generally tubular outer casing, a hypodermic needle or syringe at one end of the casing, and a plunger that slides within the casing to force liquid through the syringe. When the plunger reaches the end of the compression stroke, it activates a mechanism that releases the syringe, and a spring forces the syringe into a cavity located within the plunger. WO 2017/079811 A1 discloses an example of a safety syringe. The safety syringe disclosed in WO 2017/079811 A1 is an auto-retractable syringe having a barrel and a plunger wherein engagement of the plunger with an otherwise immobilized needle hub allows a biasing member to push the needle hub into a cavity in the plunger.

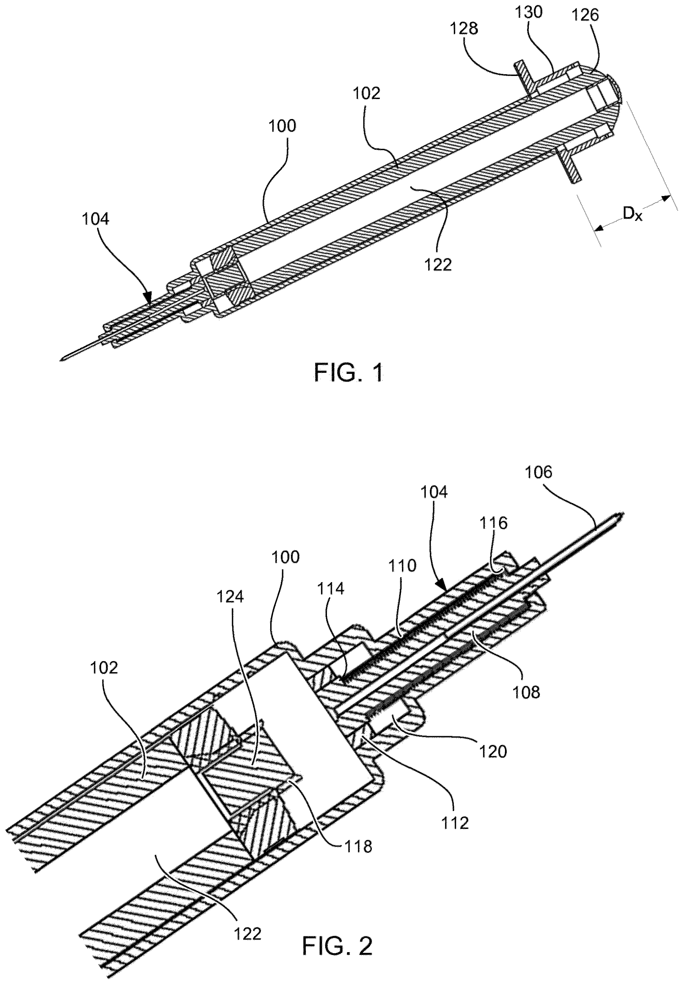

Figures 1 through 3 illustrate one known safety syringe having an outer casing 100, a plunger 102, and a retraction syringe assembly 104 located within a distal end of the casing 100. The retraction syringe assembly 104 includes a needle 106 fixed to a needle seat 108, a retraction spring 110, and a fixing sleeve 112. The spring 110 is provided in a compressed state between a forward-facing shoulder 114 on the needle seat 108, and a rearward-facing shoulder 116 on the casing 100. The fixing sleeve 112 comprises an annular ring of elastic material, which is fitted in a compressed state between an outer wall of the needle seat 108 and an inner wall of the casing 100. The fixing sleeve 112 is configured to generate a frictional force that prevents the spring 110 from expanding and thus holds the needle 106 and needle seat 108 at a fixed position with the needle 106 protruding from the distal end of the casing 100.

The plunger 102 is configured to move the fixing sleeve 112, thereby releasing the needle seat 108 from frictional engagement with the casing 100 and allowing the spring 110 to move the needle seat 108 and needle 106 to a retracted position. In particular, the plunger 102 has a forward-facing annular protrusion 118 that is sized to press against the
annular fixing sleeve 112 when the plunger 102 approaches the distal end of the casing 100. Upon application of sufficient force on the plunger 102, the protrusion 118 pushes the fixing sleeve into an annular space 120 where it no longer contacts the needle seat 108. This releases the needle seat 108 and needle 106 from frictional engagement with the casing 100.

The plunger 102 has an internal lumen 122 that is dimensioned to receive the needle seat 108 and needle 106. A stopper 124 is provided at a narrow region at the distal end of the lumen 122 to seal the lumen 122 as the plunger 102 is moved to dispense liquid through the hollow needle 106. The stopper 124 is configured to contact a rear end of the needle seat 108 at approximately the same time that the annular protrusion 118 contacts the annular fixing sleeve 112. Thus, as the plunger 102 is moved forward, the stopper 124 is displaced by contact with the back of the needle seat 108, and such movement causes the stopper 124 to move out of the narrow region of the lumen 122. When the stopper 124 moves back far enough, it enters a relatively wide region of the lumen 122, and no longer provides any resistance against the force of the spring 110. Thus, the stopper 124 is released at approximately the same time that the annular fixing sleeve 112 is released, thereby allowing the spring 110 to push the needle 106 and needle seat 108 into the plunger. More details of devices like the one in FIGs. 1 through 3 are found in U.S. Pat. Nos. 6,221,055 and 6,572,584 and U.S. Publication Nos. 2013/0006190, 2015/0283329 and 2017/0239426,

The inventor has discovered that the foregoing device has certain drawbacks.

One problem is that the annular fixing sleeve 112 provides relatively little resistance to hold the needle 106 in place, and thus the device can be retracted or partially-retracted accidentally. For example, if one attempts to push the needle 106 through a rubber stopper on a medicine vial, the force necessary to pierce the stopper may exceed the retaining force provided by the fixing sleeve 112, resulting in the needle 106 retracting when one attempts to obtain medicine through a rubber stopper. With this problem, such devices have limited use as a normal syringe. The fixing sleeve 112 also may not be strong enough to resist large hydraulic pressures generated within the casing as the plunger is forced forward to dispense the medicine.

Another problem with the foregoing device is that the plunger 102 is not positively locked inside the casing 100 when the plunger 102 is in the fully compressed position. For example, as shown in FIG. 1, when the plunger 102 is fully compressed, the proximal end 126 of the plunger 102 is located within an annular cup 130 located at the proximal end of the casing 100, which prevents a user from easily removing the plunger 102. However, it is still possible for the plunger to be removed if there is insufficient friction holding the plunger 102 in this position. It has also been found that the distance D_{X} between the proximal end 126 of the plunger 102 and the operating grips 128 located on the casing 100 can be less than the recommended standards for such medical devices (see, e.g., International Organization for Standardization standard ISO7886), which can make the device less stable and less comfortable for the operator and the patient.

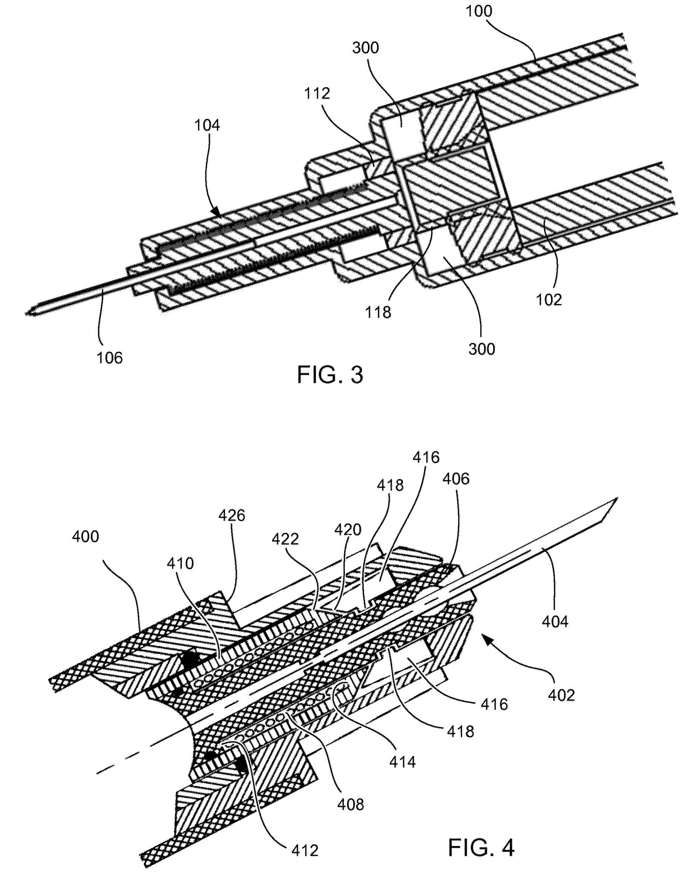

It has also been found that the device of FIGs. 1 through 3 suffers from excessively high amount of residual medicine retention when dispensing is complete. FIG. 3 illustrates this problem. When the plunger 102 is pushed forward, the annular protrusion 118 eventually contacts and seals against the annular fixing sleeve 112. At this point, medicine in the annular region 300 surrounding the annular protrusion 118 cannot pass through the needle 106. As the plunger 102 is pushed further forward, the trapped medicine moves forward around the outside of the needle seat 108, and is wasted. Some steps may be taken to mitigate this loss of medicine (e.g. forming the annular protrusion 118 with a taper or fluid passages, but some medicine will still be lost into the region surrounding the needle seat 108. The loss of even a small amount of liquid medicine can be very costly, and the losses experienced in devices such as the one in FIGs. 1 through 3 can exceed established recommendations for the minimum standard value of the residual amount of liquid medicine (see, e.g., International Organization for Standardization standard ISO7886-1).

Another known type of safety syringe device is shown in FIG. 4. This device has an outer casing 400 with a retraction syringe assembly 402 located at the distal end of the casing 400. The retraction syringe assembly 402 has a needle 404 fixed to a needle seat 406, a retraction spring 408, and a slider 410 surrounding the retraction spring 408. The spring 408 is captured in place in a compressed state between a forward-facing shoulder 412 of the needle seat 406, and a rearward-facing shoulder 414 of the slider 410, such that a restoring force of the spring 408 applies a force to move the needle seat 406 and needle 404 away from the distal end of the casing 400.

The force of the spring 408 is resisted by a plurality of hooks 416 that engage a corresponding annular groove 418 in the needle seat 406. More specifically, the hooks 416 are formed as part of the inner surface of the casing 400, and protrude into the annular groove 418. The proximal ends of the hooks 416 comprise ramps 420 that face a correspondingly-shaped conical face 422 located at the end of the slider 410. Movement of the slider 410 towards the distal end of the casing 400 causes the conical face 422 to press against the ramps 420, which moves the hooks 416 away from the annular groove 418, thus releasing the needle seat 406 from the hooks 416. The slider 410 moved by a plunger (not shown) in a manner similar to that described above in relation to FIGs. 1 through 3. Thus, upon fully-compressing the plunger into the casing 400, the slider 410 is moved to disengage the hooks 416, and the needle seat 406 and needle 404 are retracted into the plunger by the spring 408. More details of the operation of this device are available in published international application WO 2017/079811,

The inventor has discovered that the device shown in FIG. 4 also has certain drawbacks.

First, the device in FIG. 4 uses hooks 416 that are located internally within the retraction syringe assembly 402. This presents a problem because the hooks 416 must be precisely made to provide consistent release properties. Considering the shape and size of safety syringe devices, this is a relatively difficult task, because manufacturing the internal hooks 416 requires complicated molding technology and may require a combination of molding and machining technology, making their manufacture relatively expensive. For example, it may be impossible to make the internal hooks 416 directly in the outer casing 400, thus requiring the hooks to be made in a separate sub-structure 424 that is attached to the casing 400. The use of a separate sub-structure 424 increases cost, and requires additional assembly steps. This multi-part construction also is subject to manufacturing tolerance stacking problems that can affect the reliability of the connection, leading to potential leaking and safety issues, and inconsistent release force requirements between different examples of the product.

Another problem with the device of FIG. 4 is that the internal location of the hooks 416 makes it impractical or impossible to perform conventional quality control procedures to ensure that the commercial products are properly constructed with the correct dimensions and shapes necessary to provide the desired release properties. Thus, quality control is more complicated and potentially less effective.

It is also believed that the internal hook design in FIG. 4 leads to production problems, particularly in an inability to accurately and consistently make a product that matches the drawing design. It is believed that these problems result the locking force between the hooks 416 and annular groove 418 being relatively small, such that the hooks 416 can be forced out of the annular groove 418 by pressing too hard on the end of the needle 404. This can cause the retraction syringe assembly 402 to accidentally retract when pushing the needle 404 into a rubber stopper enclosing a conventional medicine vial. With this problem, such devices have limited use as a normal syringe.

It is also believed that devices such as the one shown in FIG. 4 can fail to satisfy the minimum standard value of the residual amount of liquid medicine (see, e.g., International Organization for Standardization standard ISO7886-1).

This description of the background is provided to assist with an understanding of the following explanations of exemplary embodiments, and is not an admission that any or all of this background information is necessarily prior art.

### SUMMARY

The above stated problems are addressed by the safety syringe according to the independent claim. Advantageous embodiments of the safety syringe are set forth in the dependent claims.

### BRIEF DESCRIPTION OF THE DRAWINGS

Embodiments of inventions will now be described, strictly by way of example, with reference to the accompanying drawings, in which:
FIG. 1 is a cutaway view of a prior art syringe.
FIG. 2 is a cutaway detail view of the syringe of FIG. 1.
FIG. 3 is another cutaway detail view of the syringe of FIG. 1
FIG. 4 is a cutaway view of another prior art syringe.
FIG. 5 is an isometric view of a safety syringe incorporating aspects of the present inventions.
FIG. 6 is an exploded view of the safety syringe of FIG. 5.
FIG. 7A is a cutaway detail view of the safety syringe of FIG. 5.
FIG. 7B is a cutaway detail view of an alternative embodiment of the safety syringe of FIG. 5.
FIG. 8 is a cutaway view of the safety syringe of FIG. 5, shown with the plunger assembly in a first position and the retraction syringe assembly in the starting position.
FIG. 9 is a cutaway view of the safety syringe of FIG. 5, shown with the plunger assembly in a third position and the retraction syringe assembly in the activation position.
FIG. 10 is a cutaway detail view of the safety syringe of FIG. 5, shown with the plunger assembly in the second position and the retraction syringe assembly in the starting position.
FIG. 11 is a cutaway detail view of the safety syringe assembly of FIG. 5, shown with the plug in the installed position.
FIG. 12 is a cutaway detail view of the safety syringe assembly of FIG. 5, shown with the plug removed.
FIG. 13 is a cutaway detail view of the safety syringe assembly of FIG. 5, shown with the plunger approaching the locked position.
FIG. 14 is a cutaway detail view of the safety syringe assembly of FIG. 5, shown with the plunger in the locked position.

### DESCRIPTION OF EXEMPLARY EMBODIMENTS

Aspects of the invention are now described in detail. It will be appreciated that embodiments of the invention may mitigate or alleviate all or some of the problems identified above with prior devices, but this is not strictly necessary. Indeed, the inventions described herein have merit separately and independently of any comparison with the commercial products discussed above.

As used herein, the term "proximal" refers to locations of a part that are closer to the operator of a safety syringe during use (i.e., towards the operator), and the term "distal" refers to locations of a part that are closer to the patient during use (i.e., towards the patient). The terms "axial" and "longitudinal" refer to the direction of the safety syringe from the proximal end to the distal end. "Radial" refers to the direction perpendicular to the axial direction.

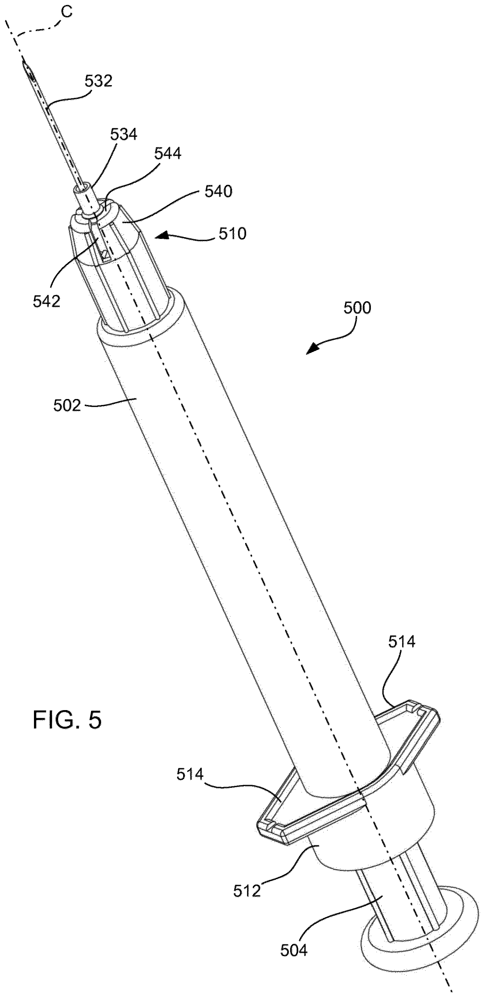

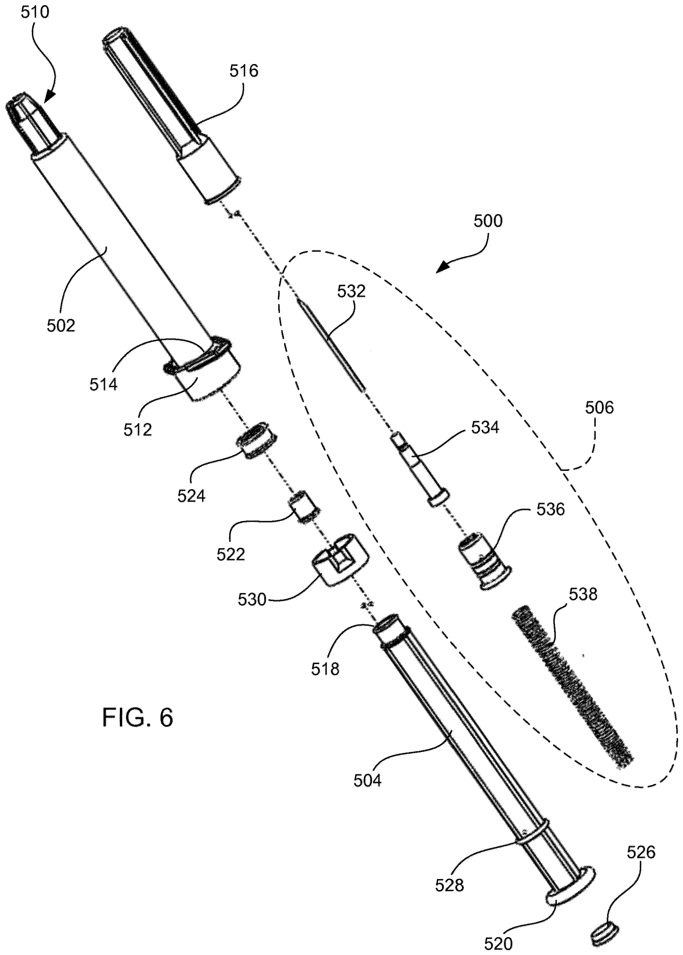

Referring now to FIGs. 5 and 6, an exemplary embodiment of a safety syringe 500 is described in detail. In general terms, the safety syringe 500 has a casing 502, a plunger 504 that fits within the casing 502, and a retraction syringe assembly 506 that is fitted to the casing 502. The casing 502 comprises a hollow channel formed by an outer surface 502' and an inner surface 502" that extend along a central axis C from a proximal end 508 to a distal end 510. At the proximal end 508, the casing 502 has an annular cup 512 that opens into the hollow channel to receive the plunger 504. The proximal end 508 also includes one or more laterally-extending grips 514 that receive the operator's finger or fingers during use. The retraction syringe assembly is positioned at the distal casing end 510, as described in more detail below. The safety syringe 500 also may include a cap 516 that selectively connects to the distal end 510 of the casing 502, such as known in the art.

The plunger 504 comprises a generally tubular hollow body that extends relative to the axis C from a distal end 518 to a proximal end 520. The plunger 504 preferably is assembled into a plunger assembly that includes the plunger 504, a plug 522 and a seal 524. The plug 522 is fitted into the plunger's hollow body at the open distal end 518, and the seal 524, which may be an annular seal or one or more O-rings or the like, is fitted to the outer perimeter of the distal end 518. The seal 524 is dimensioned to seal and slide on the inner surface of the casing 502, and the plug 522 and seal 524 together prevent liquid within the casing 502 from passing in the proximal direction through or around the plunger 504, as the plunger 504 is pushed distally towards the distal end 510 of the casing 502. A cover 526 may be provided over the proximal end 520 of the plunger 504.

The plunger 504 may include one or more radial protrusions 528 at a location near the proximal end 520. The illustrated radial protrusion 528 comprises an annular ring that extends radially from the plunger's outer surface, but other embodiments may use one or more discrete bumps, ribs, or the like. The radial protrusion cooperates with a lock 530 that is secured within the annular cup 512 of the casing 502, as explained in more detail below.

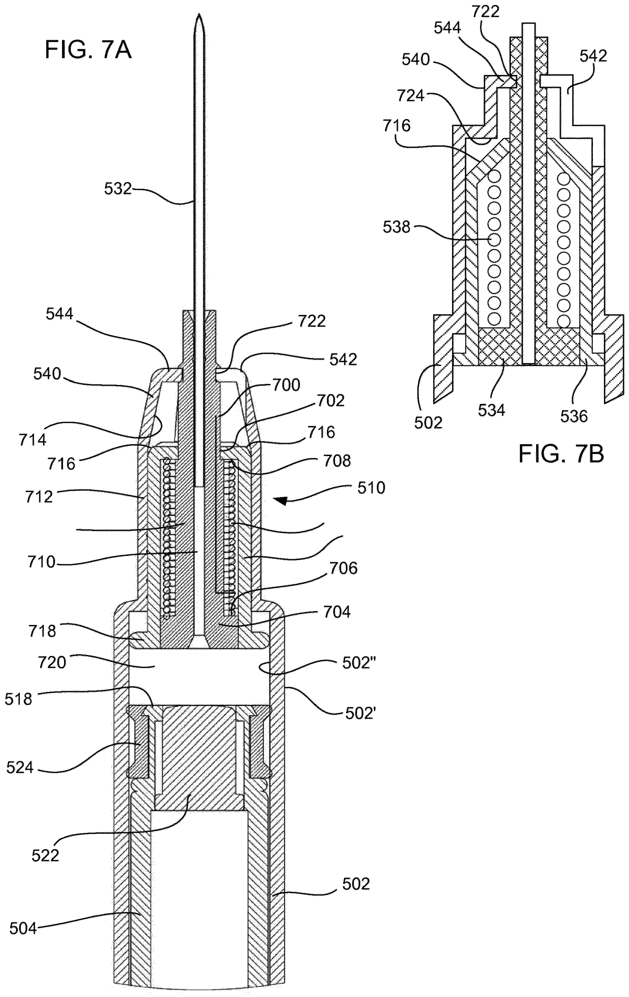

Referring now also to FIG. 7A, the connection of the retraction syringe assembly 506 to the casing 502 is described in more detail. The retraction syringe assembly 506 comprises a hollow needle 532, a needle seat 534, a slider 536, and a spring 538. The needle 532 is affixed to the needle seat 534, such as by a friction fit or adhesive bond. The distal end of the needle 532 is sharpened, and the needle 532 and needle seat 534 have a continuous internal cannula 710 for passing liquid medicine therethrough, as known in the art. The needle seat 534 is dimensioned to slide within the slider 536. To this end, a distal portion 700 of the needle seat 534 may have a first diameter that slidingly fits within a distal opening 702 of the slider 536, and a proximal portion 704 having a second, and larger, diameter that slidingly fits within a main body of the slider 536. The spring 538 is captured in place, in a compressed state, between a distally-facing shoulder 706 of the needle seat 534, and a proximally-facing shoulder 708 of the slider 536. Thus, the spring 538 generates a restoring force that tends to push the needle seat 534 and the attached needle 532 away from the slider 536 in the proximal direction. The spring 538 preferably comprises a coil spring made of material that will retain a restoring force during long periods of storage in a compressed state, such as conventional spring steel or stainless steel.

The slider 536 is located inside the distal end 510 of the casing 502. In the shown embodiment, the distal end 510 of the casing 502 includes a linear inner surface portion 712 and a tapered inner surface portion 714 extending distally from the linear inner surface portion 712. In a preferred embodiment, the linear inner surface portion 712 and the tapered inner surface portion 714 have circular cross-sectional shapes as viewed along the center axis of the casing 502, in which case the linear inner surface portion 712 is cylindrical, and the tapered inner surface portion 714 is conical. However, this is not strictly required, and other embodiments may have square, rectangular, hexagonal or other cross-sectional shapes. The tapered inner surface portion 714 reduces in axial size (i.e., reduces in distance from the center axis C of the casing 502) as it extends in the distal direction. For example, the tapered inner surface portion 714 may comprise a frustoconical shape that has a relatively large diameter at its proximal end where it joins the linear inner surface portion 712, and a relatively small diameter at its distal end. The tapered inner surface portion 714 preferably has a conic shape as viewed perpendicular to longitudinal axis of the casing 502 (see, e.g., FIG. 7A), but other shapes may be used, such as spherical shapes, parabolic shapes, and so on.

The linear inner surface portion 712 preferably is located at the distal end of a liquid medicine chamber 720, which may have a larger diameter than the linear inner surface portion 712, but this is not strictly required.

The portion of the distal end 510 of the casing 502 at the tapered inner surface portion 714 is divided into fingers 540 (Figure 5) by one or more slots 542 that extend proximally from the distal-most tip of the casing 502. The slots 542 extend through the exterior surface 502' and interior surface 502" of the casing 502, and each finger 540 includes a portion of the tapered inner surface portion 714. The fingers 540 and slots 542 are visible and accessible from the outside of the casing 502.

The slots 542 are shown as being relatively narrow as compared to the size of the fingers, 540, but this is not necessary in all embodiments. For example, the fingers 540 may comprise narrow extensions, with relatively wide slots 542 between adjacent fingers 540. Each finger 540 extends in the distal direction to a hook 544 that extends radially towards the central axis C of the casing 502. In preferred embodiments, there are three or four fingers 540, but other numbers may be used.

The slider 536 has an outer wall that is dimensioned to slide within the linear inner surface portion 712. For example, the slider 536 may have a cylindrical outer wall that fits closely to the linear inner surface portion 712 that allows sliding movement along the axis of the casing 502, but prevents lateral movements. The slider 536 has an activation surface 716 located at or near the distal end of the slider 536. When the slider 536 is in a starting position, such as shown in FIG. 7A, the activation surface 716 faces the tapered inner surface portion 714 of the casing 502, and is spaced from or in light contact with the tapered inner surface portion 714.

The needle 532, needle seat 534, slider 536 and spring 538 are installed in the casing 502 with the spring 538 compressed and the needle seat 534 extending partially from the distal end 510 of the casing 502. At the junction of the needle seat 534 and the distal end 510 of the casing 502, the distal end 700 of the needle seat 534 includes one or more radial openings 722 that are positioned to receive the one or more hooks 544. For example, the needle seat 534 may comprise openings 722 in the form of one or more annular grooves 722 that extend partially or entirely around the circumference of the needle seat 534. With the hooks 544 in the groove 722, the hooks 544 prevent the needle seat 534 from moving in the proximal direction. Thus, the hooks 544 hold the needle seat 534 and the attached needle 532 in place against the biasing force of the spring 538.

The hooks 544 are disengaged from the groove 722 by moving the slider 536 in the distal direction from a first slider position to a second slider position. Such movement causes the activation surface 716 to press against the tapered inner surface portion 714 to spread apart the fingers 540 and the attached hooks 544. As the slider 536 moves, the hooks 544 move radially away from the needle seat 534. Such movement eventually causes the hooks 544 to retract from the groove 722, at which point the hooks 544 no longer oppose the biasing force of the spring 538.

It will be appreciated that the activation surface 716 and the inner surfaces of the fingers 540 may have any suitable cooperating shapes that are functional to drive the fingers 540 away from the needle seat 534. For example, in the shown embodiment, the activation surface 716 comprises an annular surface that extends around the entire distal end of the slider 536, which slides on the tapered inner surfaces of the fingers 540. The annular surface may be replaced by discrete protrusions that align with respective ones of the fingers 540. The activation surface 716 also may be located at the distal end of the slider 536, or it may be offset in the proximal direction from the distal end. The activation surface 716 may comprise the outer rim of a distal wall that extends perpendicular to the axis of the slider 536, such as shown, or it may comprise a conical surface, a hemispherical surface, and so on, provided it is configured to press the fingers 540 outwardly as the slider 536 is advanced in the distal direction.

It is also envisioned that the activation surface and the inner surfaces of the fingers 540 may have other alternative shapes that cooperate to drive the hooks 544 out of the radial opening(s) 722. For example, FIG. 7B illustrates an alternative embodiment in which the activation surface 716 comprises a surface that tapers inwards in the distal direction (e.g., conical, hemispherical, etc.), and the fingers 540 comprise inward radial steps 724. Movement of the tapered activation surface 716 against the radial inward step 724 causes the fingers 540 to flex radially outward to release the hooks 544 from the radial openings 722. In still other embodiments, the inward radial steps 724 may be replaced by curved surfaces, tapered surface, or the like. It will also be understood that the activation surface 716 may act on any part of the fingers 540. For example, the activation surface 716 may act on a portion of the inner surface of the fingers 540 that is spaced from the hooks 544 (such as shown) or at a portion of the inner surface that is located adjacent the hooks 544. Other alternatives and variations will be apparent to persons of ordinary skill in the art in view of the present disclosure.

The distal movement of the slider 536 is achieved by pressing the plunger 504 against the slider 536. To this end, the proximal end 718 of the slider 536 extends from the linear inner surface portion 712 into the liquid medicine chamber 720, where it can be contacted by the distal end 518 of the plunger 504. As shown in FIG. 7A, the distal end 518 of the plunger 504 comprises a distally-facing surface that is configured to contact a proximal end 718 of the slider 536. In particular, the distal end 518 of the plunger 504 is shaped to contact the slider 536, and the plug 522 is positioned to contact the needle seat 534. For example, the distal end 518 of the plunger 504 may comprise an annular ring that is shaped to contact a similarly-shaped proximal end of the slider 534, without contacting the needle seat 534.

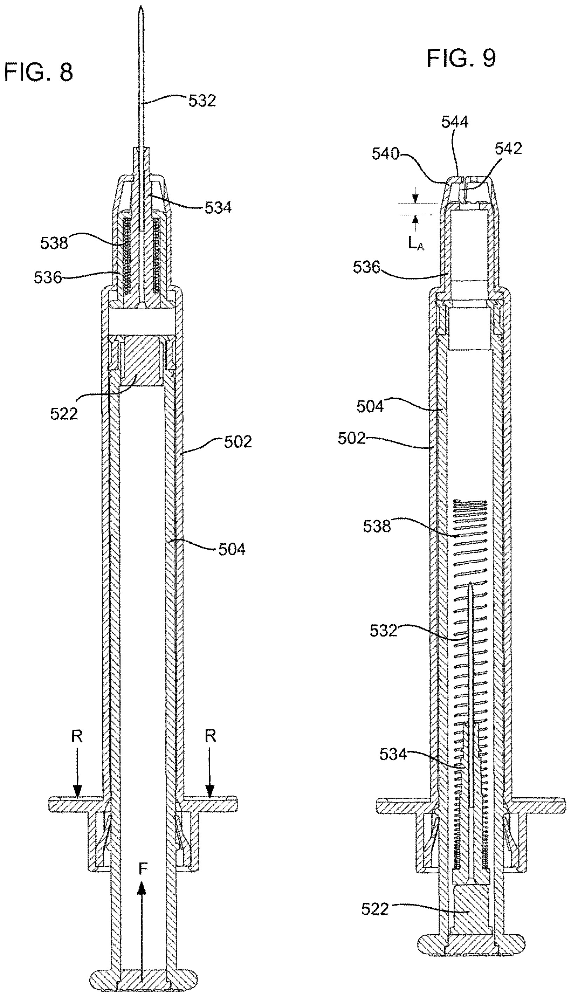

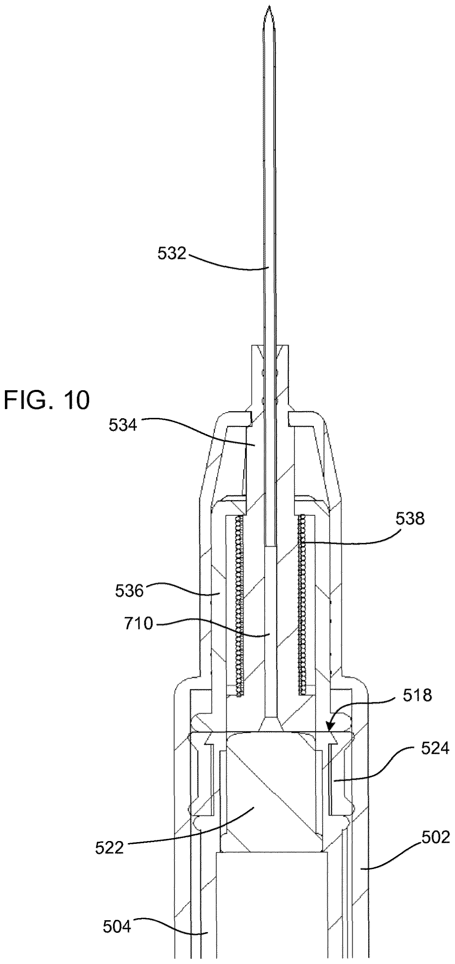

Figures 8 and 9 illustrate the operation of the retraction syringe assembly 506 in more detail. In FIG. 8, the plunger assembly (i.e., the plunger 504, plug 522 and seal 524) begins in a first position in which liquid medicine is held in the chamber 720. A force F is applied to move the plunger assembly distally relative to the casing 502 towards the retraction syringe assembly 506, and an opposite force R is applied against the grips 514 to hold the casing 502 in place. When the plunger assembly reaches a second position, the distal end 518 of the plunger 504 contacts the retraction syringe assembly 506, and particularly the slider 536 (see FIG. 10). Then the plunger assembly (or portions thereof other than the plug 522) moves distally to a third position in which the plunger 504 displaces the slider 536 distally far enough to cause the fingers 540 move far enough to pull the hooks 544 completely out of the annular groove 722. During this transitional period between the second and third plunger assembly positions, the hooks 544 prevent the needle seat 534 from moving distally along with the slider 536, and so the needle seat 534 remains in place as the plunger 504 is advanced distally. As this happens, the needle seat 534 protrudes in the proximal direction from the slider 536 to press against the plug 522. After a certain amount of this movement, the plug 522 will be loosened from its frictional engagement with the inside of the plunger 504. As the plunger 504 continues to move the slider 536 distally, the hooks 544 eventually release from the annular groove 722, at which time the spring 538 pushes the needle seat 534 and needle 532, as well as the loosened or released plug 522, in the proximal direction to retract them safely inside the plunger 504, as shown in FIG. 10. Thus, it will be understood that movement of the plunger 504 in the distal direction causes the hooks 544 to release the annular groove 722, and causes the plug 522 to release from the plunger 504, which allow the spring 538 to retract the needle 532 entirely into the safety syringe 500.

The foregoing configuration is expected to provide several advantages over conventional syringe retraction mechanisms. First, the construction of the fingers 540 and hooks 544 by providing slots 542 at the distal end 510 of the syringe 500 is significantly more easily accomplished than forming a retaining mechanism that is enclosed within the syringe casing, such as in the device in shown in FIG. 4 and described in WO 2017/079811. In particular, the fingers 540, slots 542 and hooks 544 can be formed using a simple two-part injection molding apparatus. Second, providing the fingers 540 and hooks 544 as part of the exterior of the casing 502 allows the quality of the manufacturing process to be easily monitored. Third, if the hooks 544 fail to release from the annular groove 722, the operator can manually retract the hooks 544 to force the needle 532 to retract (e.g., by inserting an object into the slots 542 and prying the fingers 540 apart). Fourth, the safety syringe 500 can be examined by the final user to ensure that the hooks 544 are properly inserted in the annular groove 722 before using the syringe 500, which improves the safety of the device.

It is also noted that the hooks 544 of the exemplary embodiment may be formed at the ends of fingers 540 that have (at least at one location along their length) a major axis that is perpendicular to the direction in which the hooks 544 must move to disengage from the annular groove 722, and a minor axis that is parallel to the movement direction (the required movement direction being the radial direction away from the annular groove 722). Thus, the fingers 540 exhibit relatively little resistance to bending in the direction necessary to release the hooks 544 from the annular groove 722, but still provide a strong connection between the hooks 544 and the annular groove 722 to prevent accidental retraction. Thus, embodiments such as the exemplary embodiment can be used in the same way as a conventional syringe. For example, embodiments can be used to pierce a rubber stopper of a conventional medicine vial without causing the needle 532 to accidentally retract.

It will be appreciated from this disclosure that the exact shape of the fingers 540 can be modified to change the flexing properties of the fingers 540 to fine-tune the ergonomic operation of the device. For example, making the fingers 540 thicker in the axial direction can increase the amount of force necessary to disengage the hooks 544, and vice-versa. The fingers 540 also may include narrowed portions that act as "living hinges" to provide a more discrete location at which the fingers 540 pivot. Other alternatives and variations will be apparent to persons of ordinary skill in the art in view of the present disclosure. This allows the safety syringe 500 to be configured to provide smooth operation without significant changes in the amount of force necessary to slide the plunger 504 or activate the retraction syringe assembly 506, which improves the overall ergonomic performance.

In contrast, the device shown in FIG. 4 and WO 2017/079811 has retaining mechanisms that extend with their major axis parallel to the release direction, which means a relatively large force is inherently necessary to release the needle seat. Such force can make the syringe less ergonomic to use, and it is believed that manufacturers of such devices have resorted to making the retaining mechanisms particularly thin to reduce the operation force, which makes the part harder to quality control, more prone to accidental release, and less reliable. Such devices are not suitable for use as a conventional syringe because they have difficulty piercing rubber medicine vial stoppers without accidental retraction.

Also, by comparison, the device shown in FIGs. 1 through 3 uses a fixing sleeve 112 to hold the needle in place, which limits the functionality of such devices. In particular, the fixing sleeve is not able to resist large forces such as those generated when piercing rubber medicine vial stoppers, without also making the fixing sleeve particularly difficult to displace during normal use. Thus, such devices sacrifice either ergonomics (i.e., smooth and low-force operation), or the ability to operate as a conventional syringe to pierce rubber stoppers and the like.

In a preferred embodiment, the proximal end of the retraction syringe assembly 506 and the distal end of the plunger assembly are shaped as generally matching flat surfaces that extend across the entire cross-section of the liquid medicine chamber 720. For example, in the shown embodiment, the proximal end of the slider 536 is flat and has a radially-extending portion that extends to the inner wall of the chamber 720, such that the slider 536 has a T-shape, and the needle seat 534 is flat and flush with the slider 536, so that the proximal end of the assembled parts forms a continuous flat surface across the chamber 720 (except for an opening into the cannula 710) that extends in a plane that is perpendicular to the central axis C. Similarly, the distal ends of the plunger 504, the plug 522, and the seal 524 all are generally flat and lie in a common plane that extends perpendicular to the central axis C, to also form a continuous surface that extends across the entire chamber 720 (the seal 524 may protrude from the plunger 504 slightly in the distal direction to ensure proper sealing against the slider 536). As shown in FIG. 10, this configuration minimizes the amount of volume in the chamber 720 when the plunger 504 engages the slider 536, thereby minimizing the loss of liquid medicine during injection. It will also be understood that the use of flat matching surfaces provides the lowest possible volume of free space between the surfaces, particularly as compared to matching convex and concave shapes, which can allow a greater volume of lost fluid.

As indicated above, it is expected that the ergonomic quality of the safety syringe 500 can be improved by reducing the amount of work necessary to cause the retraction syringe assembly 506 to activate. One aspect of the operation relates to the forces necessary to release the plug 544 from the plunger 504 to allow the needle seat 534 and needle 532 to retract. In conventional devices (see, e.g., U.S. Pat. No. 6,572,584, a plug (42) is retained in the plunger (32) at a widened portion at the proximal end of the plug, while the distal end of the plug protrudes distally and out of contact with the plunger. In such a device, the plug is frictionally engaged with the plunger along a continuous length of the plug. Thus, the plug must be continuously pushed along the entire length of the frictional connection region before the plug is released, which requires more work and can make the syringe relatively difficult to operate.

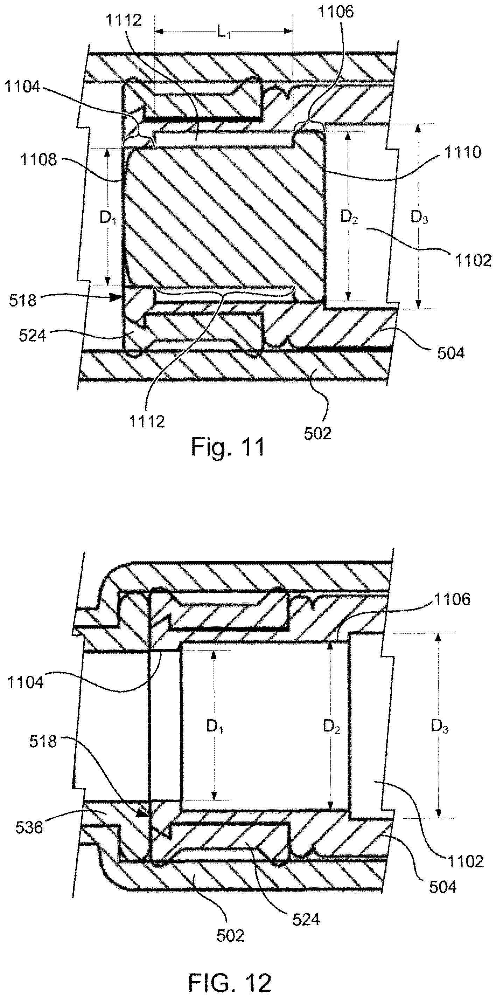

To address this problem, embodiments may include a stepped connection between the plug 522 and the plunger 504, such as shown in FIGs. 11 and 12. In this exemplary embodiment, the inner passage 1102 of the plunger 504 has a first region 1104 at the distal end of the plunger 518 having a first diameter D₁, and a second region 1106 located a predetermined length L₁ in the proximal direction from the first region 1104 and having a second diameter D₂. The second diameter D₂ is greater than the first diameter D₁. The inner passage 1102 expands to a third diameter D₃ at the proximal end of the second region 1106, with the third diameter D₃ being greater than the second diameter D₂. Thus, the inner passage 1102 has three "steps," with each step having a larger diameter than the adjacent distal step. These steps may be discrete (i.e., formed by walls that extend perpendicular to the central axis, or they may have tapers or other shapes).

Similarly, the plug 522 has a distal end 1108 with an outer diameter that frictionally engages the first diameter D₁, and a proximal end 1110 with an outer diameter that frictionally engages the second diameter D₂. To obtain frictional engagement, the plug 522 may comprise an elastomeric or other resilient material, and the diameters of the plug 522 at locations corresponding to the first region 1104 and second region 1106 may be slightly larger than the first and second diameters D₁, D₂, respectively, to provide a friction fit. The plug 522 also has an intermediate region 1112 located between the distal and proximal ends 1108, 1110, where the diameter of the plug 522 is less than the diameter of the adjacent portion of the passage 1102, such that there is no contact between the passage 1102 and the plug 522 within the intermediate region 1112. The intermediate region 1112 has a length in the axial direction corresponding to the length L₁ between the first region 1104 and the second region 1106 of the passage 1102. Thus, the plug 522 is frictionally connected to the passage 1102 at the distal end 1108 of the plug 522 and at the proximal end 1110 of the plug 522, but not at the intermediate region 1112.

The intermediate region 1112 can have any suitable shape. For example, in the shown embodiment, the intermediate region 1112 has a continuous diameter that is approximately equal to the first diameter D₁, and the portion of the passage 1102 that is adjacent to the plug's intermediate region 1112 has a diameter the is equal to the second diameter D₂. In other examples, other relative shapes may be used. For example, the intermediate region 1112 of the plug 522 may taper in the proximal direction from the first diameter D₁ to the second diameter D₂. As another example, the intermediate region 1112 may have a continuous diameter approximately equal to the first diameter D₁, while the adjacent passage wall 1102 tapers from the first diameter D₁ to the second diameter D₂. Other alternatives and variations will be apparent to persons of ordinary skill in the art in view of the present disclosure.

This connection is expected to provide an advantage in relation to the release work profile required to activate the retention syringe assembly 506. In a conventional device having a single connection region between the plug and the plunger, the plug must be pushed along the entire length of the connection region before it is released. In contrast, the plug 522 described above has a total connection region length equal to the sum of the length of the first region 1104 and the length of the second region 1106, but the plug 522 is fully released from frictional connection with the passage 1102 upon moving less than the total length of frictional connection regions 1104 and 1106. For example, in embodiments in which the axial lengths of the first and second regions 1104, 1106 are equal, the plug 522 will release from both regions 1104, 1106 simultaneously upon traveling a distance equal to half the total friction connection length. Thus, for a given length of the connection between the plug and the plunger, the amount of work necessary to release an embodiment such as shown in FIGs. 11 and 12 is less than (and as little as half) the amount of work that is necessary to release a conventional plug having a single continuous region of frictional contact with the plunger. This is expected to make the safety syringe 500 easier to use and to improve the patient experience.

The use of two separate frictional connections between the plug 522 and the passage 1102 such as shown in FIGs. 11 and 12 also provides greater lateral stability and reduces the likelihood that the plug 522 will be turned to block the passage 1102.

The size of the intermediate region 1112 may be selected to optimize or improve the release work profile, while also accounting for a desire to keep the plug 522 long enough to ensure that it does not twist or rotate into a jammed position during needle retraction. In a preferred embodiment, the intermediate region is equal to 50-85% of the total length of the plug 522, and more preferably 60-75% of the total length of the plug 522, and more preferably about 65-70% of the length of the plug 522. It is also preferred for the first and second connection regions 1104 and 1106 to be equal in longitudinal size (i.e., length in the axial direction), but this is not strictly required in all embodiments.

The sizes of the frictional connection regions 1104, 1106 preferably are selected such that the maximum length of one or both regions 1104, 1106 in the axial direction is equal to or less than the distance necessary for the slider 536 to move in order to disengage the hooks 544 from the annular groove 722. For example, as shown in FIG. 9, it may be necessary to move the slider 536 an activation distance L_{A} relative to the casing 502 before the hooks 544 clear the annular groove 722. It is preferable for the plug 522 to be fully released from the plunger 504 when the slider 536 reaches the activation distance L_{A}. Thus, by making the frictional connection regions 1104, 1106 with a length in the axial direction that is less than or equal to the activation distance L_{A}, it can be assured that no further distal movement of the plunger 504 relative to the casing 502 is necessary to release the plug 522 from the plunger 504.

While the foregoing feature is desirable, it will be appreciated that it is not strictly necessary in all embodiments. For example, if the axial length of one or both frictional connection regions 1104, 1106 is greater than the activation distance L_{A}, the plug 522 may be sufficiently released from the plunger 504 that any remaining connection force is overcome by the spring 538, or is easily overcome by the operator's continued application of the releasing force F.

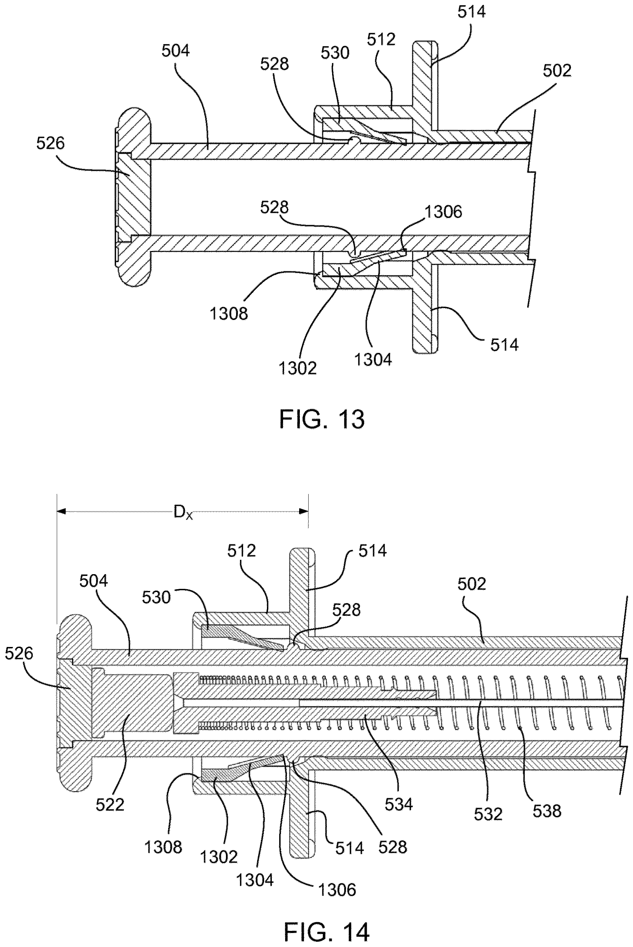

Referring now to FIGs. 13 and 14, embodiments also may include a mechanism to lock the plunger 504 after the retraction syringe assembly 506 is activated to retract the needle 532. For example, as noted above, the plunger 504 may include one or more radial protrusions 528, which are captured by a lock 530 when the plunger 504 has been moved far enough to activate the retraction syringe assembly 506. In the shown example, the lock 530 comprises an annular body 1302 that surrounds the plunger 504 and fits snugly within the annular cup 512 formed at the proximal end of the casing 502. One or more tabs 1304 extend from the annular body 1302 towards the plunger 504. The tabs 1304 are positioned to interfere with the radial protrusions 528 as the plunger 504 is moved relative to the casing 502. For example, the radial protrusions 528 may extend a first distance from the axis of the plunger 504, and the tabs 1304 may extend to tab ends 1306 that are located within the first radial distance. Thus, contact between the tabs 1304 and the radial protrusions inhibits or prevents the plunger 504 from moving relative to the casing 502.

The positions of the tab ends 1306 and the radial protrusions 528 are selected such that the distally-facing sides of the tab ends 1306 engage the proximally-facing sides of the protrusion 528 when the plunger 504 has traveled far enough in the distal direction to cause the retractions syringe assembly 506 to activate. Thus, when the needle 532 is retracted into the plunger 504, the plunger 504 cannot be retracted from the casing 502. This provides an added measure of safety by preventing operation of the plunger 504 from accidentally causing the needle 532 to become exposed. In the shown example, the tab ends 1306 and radial protrusions 528 are located to engage one another immediately upon moving the plunger assembly to the third position to cause the needle 532 to retract, and thus the lock 530 prevents the plunger assembly from moving from the third position to the second and first positions. In other cases, the lock 530 may be configured such that the plunger 504 can still be withdrawn some small amount, but not far enough to present a plausible risk of the needle 532 becoming exposed. For example, the lock 530 may be configured to allow the plunger 504 to move back to the second plunger assembly position, but not all the way to a starting position.

Any suitable configuration may be used for the tabs 1304 and radial protrusions 528. In a preferred embodiment, the tabs 1304 and protrusions 528 are configured to have different engagement properties depending on the direction in which the plunger 504 is being moved relative to the casing 502. In particular, it is desirable for the protrusions 528 to be easily moved past the tabs 1304 when the plunger 504 is moving in the distal direction relative to the casing 502, but the protrusions 528 cannot be moved past the tabs 1304 (at least not without substantial difficulty) when the plunger 504 is moved in the proximal direction relative to the casing 502. In the shown example, such differential force properties are obtained by orienting the tabs 1304 to taper towards the plunger 504 with respect to the distal direction. With this configuration, the protrusions 528 can slide along the tapered surface of the tabs 1304 to create a force having an outward radial component to gently push the tabs 1304 radially away from the plunger 504 as the plunger 504 is moved distally. However, when the plunger 504 is moved proximally, the protrusions 528 contact distal faces of the tabs 1304, such that forces between the protrusions 528 and the tabs 1304 are oriented parallel to the movement direction, thus causing the tabs 1304 to prevent further movement of the protrusions 528.

It will be appreciated that alternative configurations may be used to obtain a similar differential force profile. For example, the tabs 1304 may comprise simple radial protrusions (i.e., not tapered in the distal direction as shown), and the protrusions 528 may comprise flexible fingers that taper outward in the proximal direction. Thus, the protrusions 528 flex towards the plunger 504 to pass the tabs 1304 as the plunger 504 is moved distally, but spring back away from the plunger 504 when they pass the tabs 1304 to prevent the operator from pulling the plunger 504 proximally after the retraction syringe assembly 506 is activated. Other alternatives and variations will be apparent to persons of ordinary skill in the art in view of the present disclosure.

The lock 530 may be attached to the casing 502 using any suitable method of forming the parts. In the shown embodiment, the lock 530 is a separate part that is held in place within the annular cup 512 by an inward radial lip 1308 formed at the proximal end of the annular cup 512. In other embodiments, the lock 530 may be retained by adhesive bonding, heat staking, fasteners, friction fit, and so on. In still other embodiments, the lock 530 may be formed integrally with the casing 502. For example, the tabs 1304 may be formed monolithically with the annular cup 512, in which case the annular body 1302 may be omitted. It is also envisioned that the tabs 1304 may be attached directly to the annular cup 512 without requiring an annular body 1302 to join the tabs 1304 together. For example, one or more tabs 1304 may be heat staked or ultrasonically welded at discrete locations to the inside of the annular cup. Other alternatives and variations will be apparent to persons of ordinary skill in the art in view of the present disclosure.

The use of a lock 530 such as the one described above is also expected to provide a benefit in relation to the ergonomic operation of the safety syringe 500. In particular, such a lock allows the safety syringe 500 to be made with any desired distance D_{X} between the proximal end of the plunger 504 and the distal faces of the grips 514. For example, this distance can be selected to comply with recommended standards such as those in International Organization for Standardization standard ISO7886. Making this distance at or above a minimum predetermined distance can make the device easier to operate and more comfortable for the patient. This also allows the device to be manufactured such that the proximal end of the plunger 504 extends from the annular cup 512 after the retraction syringe assembly 506 has been activated.

In comparison, a device like the one shown in FIGs. 1 through 3 relies upon burying the proximal end 126 of the plunger 102 into the annular cup 130 to prevent later removal after use. This places the operator's thumb relatively close to the grips 128, making the device less comfortable for the operator and the patient.

It will be appreciated that embodiments of the inventions described herein may be constructed using any suitable technology and may comprise any suitable material. For example, the needle 532 and spring 538 may be metal, and the remaining parts may be formed of plastic or elastomeric materials using injection molding or other technologies. It will also be appreciated that parts may be made of any number of subassemblies. For example, the casing 502 may be manufactured as multiple parts that are attached together (e.g., the distal end 510 being made separately from the proximal end).

The present disclosure describes a number of inventive features and/or combinations of features that may be used alone or in combination with each other or in combination with other technologies. The embodiments described herein are all exemplary, and are not intended to limit the scope of the claims. It will also be appreciated that the inventions described herein can be modified and adapted in various ways, and all such modifications and adaptations are intended to be included in the scope of this disclosure.

## Claims

1. A safety syringe (500) comprising:
a casing (502) having an inner surface (502") and an outer surface (502') extending along a central axis (C) from a proximal casing end (508) to a distal casing end (510), the distal casing end (510) being divided by one or more slots extending proximally from a distal-most end of the casing (502) and through the inner surface (502") and the outer surface (502") to form a plurality of distally-extending fingers (540), each of the fingers (540) comprising a respective inner surface and a respective hook (544) that extends in a radial direction towards the central axis (C);
a plunger assembly extending along the central axis (C) and movable within a chamber (720) in the casing (502); and
a retraction syringe assembly (506) comprising:
a slider (536) having an activation surface (716) facing the plurality of distally-extending fingers (540),
a needle seat (534) configured to slide along the central axis (C) within the slider (536), the needle seat (534) having a distal portion configured to extend through a distal end of the slider (534), the distal portion having one or more radial openings (722) configured to receive the hooks (544) therein,
a hollow needle (532) attached to and configured to move with the needle seat (534), and
a spring (538) positioned between the slider (536) and the needle seat (534) and configured to bias the needle seat (534) away from the slider (536),
wherein the slider (536) is movable along the central axis (C) between a first slider position in which the activation surface (716) does not displace the hooks (544) from the one or more radial openings (722), and a second slider position in which the activation surface (716) interferes with the inner surfaces of the fingers (540) and thereby displaces the hooks (544) from the one or more radial openings (722),
**characterized in that**
the plunger assembly comprises a plunger (504) having a hollow body, a plug (522) located inside the hollow body, and a seal (524) surrounding the plunger (504) and extending to form a seal against the inner surface of the casing (502);
the distal end (518) of the plunger (504), a distal end of the plug (522), and a distal end of the seal (524) collectively form a first planar surface extending in a plane perpendicular to the central axis (C) continuously across the chamber (720); and
the proximal end of the slider (536) and the proximal end of the needle seat (534) collectively form a second planar surface extending in a plane perpendicular to the central axis (C) continuously across the chamber (720) except for an entrance to the hollow needle.

2. The safety syringe of claim 1, wherein each of the fingers (544) comprises a distally-extending tapered inner surface portion (712) that reduces in distance from the central axis (C) in the distal direction; the slider (536) has a proximally-facing shoulder (708) and the needle seat (534) has a distally-facing shoulder (706), and the spring (538) is positioned between the proximally-facing shoulder (708) and the distally-facing shoulder (706).

3. The safety syringe of claim 1, wherein the plunger (504) has an inner passage (1102) having a first region (1104) with a first radius, and a second region (1106) with a second radius, the second radius being larger than the first radius; and
wherein the plug (522) has a distal plug end (1108) frictionally engaged with the first region (1104), a proximal plug end (1110) frictionally engaged with the second region (1106), and an intermediate region (1112) located between the distal plug end (1108) and the proximal plug end (1110), wherein an outer surface of the plug (522) in the intermediate region (1112) is not in contact with the inner passage (1102).

4. The safety syringe of claim 1, wherein the plunger (504) has one or more radial protrusions (528) and the casing (502) comprises a lock (530) configured to engage the one or more radial protrusions (528) to inhibit movement of the plunger (504) towards the proximal casing end, the lock (530) comprises one or more tabs (1304) extending from the casing (502) towards the plunger (504).

## Patentansprüche

1. Eine Sicherheitsspritze (500), bestehend aus:
ein Gehäuse (502) mit einer Innenfläche (502") und einer Außenfläche (502'), die sich entlang einer Mittelachse (C) von einem proximalen Gehäuseende (508) zu einem distalen Gehäuseende (510) erstreckt, das distale Gehäuseende (510) durch einen oder mehrere Schlitze unterteilt ist, die sich proximal von einem am weitesten distal gelegenen Ende des Gehäuses (502) und durch die innere Oberfläche (502") und die äußere Oberfläche (502") erstrecken, um eine Vielzahl von sich distal erstreckenden Fingern (540) zu bilden, wobei jeder der Finger (540) eine entsprechende innere Oberfläche und einen entsprechenden Haken (544) umfasst, der sich in einer radialen Richtung zur zentralen Achse (C) hin erstreckt;
eine Kolbenanordnung, die sich entlang der Mittelachse (C) erstreckt und in einer Kammer (720) im Gehäuse (502) beweglich ist; und
eine Rückzugsspritzenanordnung (506), die Folgendes umfasst:
einen Schieber (536) mit einer Aktivierungsfläche (716), die der Vielzahl der sich nach außen erstreckenden Finger (540) zugewandt ist,
einen Nadelsitz (534), der so konfiguriert ist, dass er entlang der Mittelachse (C) innerhalb des Schiebers (536) gleitet, wobei der Nadelsitz (534) einen distalen Abschnitt aufweist, der so konfiguriert ist, dass er sich durch ein distales Ende des Schiebers (534) erstreckt, wobei der distale Abschnitt eine oder mehrere radiale Öffnungen (722) aufweist, die so konfiguriert sind, dass sie die Haken (544) darin aufnehmen,
eine Hohlnadel (532), die an dem Nadelsitz (534) befestigt und so konfiguriert ist, dass sie sich mit diesem bewegt, und
eine Feder (538), die zwischen dem Schieber (536) und dem Nadelsitz (534) angeordnet und so konfiguriert ist, dass sie den Nadelsitz (534) vom Schieber (536) weg vorspannt,
wobei der Schieber (536) entlang der Mittelachse (C) zwischen einer ersten Schieberposition, in der die Aktivierungsfläche (716) die Haken (544) nicht aus der einen oder den mehreren radialen Öffnungen (722) verschiebt, und einer zweiten Schieberposition, in der die Aktivierungsfläche (716) mit den Innenflächen der Finger (540) zusammenwirkt und dadurch die Haken (544) aus der einen oder den mehreren radialen Öffnungen (722) verschiebt, beweglich ist,
**dadurch gekennzeichnet, dass**
die Plungeranordnung einen Plunger (504) mit einem Hohlkörper, einen Stopfen (522), der sich im Inneren des Hohlkörpers befindet, und eine Dichtung (524) umfasst, die den Plunger (504) umgibt und sich so erstreckt, dass sie eine Dichtung gegen die Innenfläche des Gehäuses (502) bildet;
das distale Ende (518) des Kolbens (504), ein distales Ende des Stopfens (522) und ein distales Ende der Dichtung (524) gemeinsam eine erste ebene Fläche bilden, die sich in einer Ebene senkrecht zur Mittelachse (C) kontinuierlich über die Kammer (720) erstreckt; und
das proximale Ende des Schiebers (536) und das proximale Ende des Nadelsitzes (534) gemeinsam eine zweite ebene Fläche bilden, die sich in einer Ebene senkrecht zur Mittelachse (C) durchgehend über die Kammer (720) mit Ausnahme eines Eingangs zur Hohlnadel erstreckt.

2. Sicherheitsspritze nach Anspruch 1, wobei jeder der Finger (544) einen sich nach distal erstreckenden, sich verjüngenden inneren Oberflächenabschnitt (712) aufweist, der sich in distaler Richtung in seinem Abstand von der Mittelachse (C) verringert; der Schieber (536) eine nach proximal weisende Schulter (708) und der Nadelsitz (534) eine nach distal weisende Schulter (706) aufweist, und die Feder (538) zwischen der nach proximal weisenden Schulter (708) und der nach distal weisenden Schulter (706) angeordnet ist.

3. die Sicherheitsspritze nach Anspruch 1, wobei der Kolben (504) einen inneren Durchgang (1102) mit einem ersten Bereich (1104) mit einem ersten Radius und einem zweiten Bereich (1106) mit einem zweiten Radius aufweist, wobei der zweite Radius größer als der erste Radius ist; und
wobei der Stopfen (522) ein distales Stopfenende (1108), das reibschlüssig mit dem ersten Bereich (1104) in Eingriff steht, ein proximales Stopfenende (1110), das reibschlüssig mit dem zweiten Bereich (1106) in Eingriff steht, und einen Zwischenbereich (1112) aufweist, der zwischen dem distalen Stopfenende (1108) und dem proximalen Stopfenende (1110) angeordnet ist, wobei eine Außenfläche des Stopfens (522) in dem Zwischenbereich (1112) nicht in Kontakt mit dem inneren Durchgang (1102) steht.

4. Sicherheitsspritze nach Anspruch 1, wobei der Kolben (504) einen oder mehrere radiale Vorsprünge (528) aufweist und das Gehäuse (502) eine Verriegelung (530) umfasst, die so konfiguriert ist, dass sie mit dem einen oder den mehreren radialen Vorsprüngen (528) in Eingriff kommt, um eine Bewegung des Kolbens (504) in Richtung des proximalen Gehäuseendes zu verhindern, wobei die Verriegelung (530) eine oder mehrere Laschen (1304) umfasst, die sich vom Gehäuse (502) in Richtung des Kolbens (504) erstrecken.

## Revendications

1. Seringue de sécurité (500) comprenant :
une enveloppe (502) ayant une surface intérieure (502") et une surface extérieure (502') s'étendant le long d'un axe central (C) d'une extrémité proximale de l'enveloppe (508) à une extrémité distale de l'enveloppe (510), l'extrémité distale du boîtier (510) est divisée par une ou plusieurs fentes s'étendant proximalement à partir d'une extrémité la plus distale du boîtier (502) et à travers la surface intérieure (502") et la surface extérieure (502") pour former une pluralité de doigts d'extension distale (540), chacun des doigts (540) comprenant une surface intérieure respective et un crochet respectif (544) qui s'étend dans une direction radiale vers l'axe central (C) ;
un ensemble plongeur s'étendant le long de l'axe central (C) et mobile à l'intérieur d'une chambre (720) dans le boîtier (502) ; et
un ensemble de seringue de rétraction (506) comprenant :
un curseur (536) ayant une surface d'activation (716) faisant face à la pluralité de doigts à extension distale (540),
un siège d'aiguille (534) configuré pour glisser le long de l'axe central (C) à l'intérieur du curseur (536), le siège d'aiguille (534) ayant une partie distale configurée pour s'étendre à travers une extrémité distale du curseur (534), la partie distale ayant une ou plusieurs ouvertures radiales (722) configurées pour recevoir les crochets (544) à l'intérieur,
une aiguille creuse (532) fixée au siège de l'aiguille (534) et configurée pour se déplacer avec lui, et
un ressort (538) placé entre le curseur (536) et le siège de l'aiguille (534) et configuré pour écarter le siège de l'aiguille (534) du curseur (536),
dans lequel le curseur (536) est mobile le long de l'axe central (C) entre une première position du curseur dans laquelle la surface d'activation (716) ne déplace pas les crochets (544) des une ou plusieurs ouvertures radiales (722), et une deuxième position du curseur dans laquelle la surface d'activation (716) interfère avec les surfaces intérieures des doigts (540) et déplace ainsi les crochets (544) des une ou plusieurs ouvertures radiales (722),
**caractérisé par le fait que**
l'ensemble plongeur comprend un plongeur (504) ayant un corps creux, un bouchon (522) situé à l'intérieur du corps creux, et un joint (524) entourant le plongeur (504) et s'étendant pour former un joint contre la surface intérieure du boîtier (502) ;
l'extrémité distale (518) du piston (504), l'extrémité distale du bouchon (522) et l'extrémité distale du joint (524) forment collectivement une première surface plane s'étendant dans un plan perpendiculaire à l'axe central (C) de manière continue à travers la chambre (720) ; et
l'extrémité proximale du curseur (536) et l'extrémité proximale du siège de l'aiguille (534) forment collectivement une seconde surface plane s'étendant dans un plan perpendiculaire à l'axe central (C) de façon continue à travers la chambre (720), à l'exception de l'entrée de l'aiguille creuse.

2. La seringue de sécurité de la revendication 1, dans laquelle chacun des doigts (544) comprend une partie de surface intérieure conique (712) qui s'étend distalement et qui diminue en distance de l'axe central (C) dans la direction distale ; le curseur (536) a un épaulement (708) orienté vers le haut et le siège de l'aiguille (534) a un épaulement (706) orienté vers le bas, et le ressort (538) est positionné entre l'épaulement (708) orienté vers le haut et l'épaulement (706) orienté vers le bas.

3. la seringue de sécurité de la revendication 1, dans laquelle le piston (504) a un passage intérieur (1102) ayant une première région (1104) avec un premier rayon, et une deuxième région (1106) avec un deuxième rayon, le deuxième rayon étant plus grand que le premier rayon ; et
dans lequel le bouchon (522) a une extrémité distale (1108) en prise par friction avec la première région (1104), une extrémité proximale (1110) en prise par friction avec la deuxième région (1106), et une région intermédiaire (1112) située entre l'extrémité distale (1108) et l'extrémité proximale (1110), dans laquelle une surface extérieure du bouchon (522) dans la région intermédiaire (1112) n'est pas en contact avec le passage intérieur (1102).

4. La seringue de sécurité de la revendication 1, dans laquelle le piston (504) a une ou plusieurs protubérances radiales (528) et le boîtier (502) comprend un verrou (530) configuré pour engager la ou les protubérances radiales (528) afin d'empêcher le mouvement du piston (504) vers l'extrémité proximale du boîtier, le verrou (530) comprend une ou plusieurs languettes (1304) qui s'étendent du boîtier (502) vers le piston (504).
